(19) Europäisches Patentamt
European Patent Office
Office européen des brevets

(11) EP 1 051 911 A1

# (12) EUROPEAN PATENT APPLICATION

(43) Date of publication:
**15.11.2000 Bulletin 2000/46**

(51) Int. Cl.[7]: **A01N 43/86**

(21) Application number: **00303665.4**

(22) Date of filing: **02.05.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **08.05.1999 GB 9910545**

(71) Applicant: **Sorex Limited**
**Widnes, Cheshire WA8 8TJ (GB)**

(72) Inventors:
- **Hodgkinson, Leslie Charles Dr.**
  **Runcorn, Cheshire WA7 1ES (GB)**
- **LeQuesne, Lesley Carol**
  **Bridleways, Widnes, Cheshire WA8 9AT (GB)**

(74) Representative:
**Wilkinson, Stephen John**
**Stevens, Hewlett & Perkins**
**1 St. Augustine's Place**
**Bristol BS1 4UD (GB)**

## (54) Control of acarids

(57) The compound 1-keto-3-(2-fur-2-ylethenyl)2-oxodihydroquinoline (I) has acaricidal properties and has use in a method of combating acarids, particularly *Psoroptes cuniculi* and *Psoroptes ovis*. Acaricidal compounds comprise compound I together with an inert carrier.

EP 1 051 911 A1

## Description

The present invention relates to the treatment of acarids using the compound 1-keto-3-(2-fur-2-ylethenyl)2-oxodihydroquinoline. More particularly, it relates to a method of treating acarids using this compound and to compositions containing it.

The invention provides the use of 1-keto-3-(2-fur-2-ylethenyl)2-oxodihydroquinoline (1) as an acaricide. According to a further aspect, it provides a method of combating acarids which comprises treating them with an acaricidally-effective amount of the compound 1. According to yet a further aspect, the invention provides an acaricidal composition comprising an inert carrier and, as active ingredient, the compound 1.

The compound 1, identified above, which has the structural formula

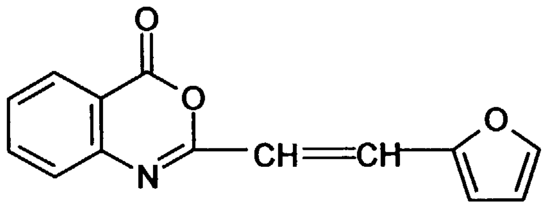

has been found to exhibit acarcidal activity against the ectoparasitic mites _Psoroptes_ _cuniculi_ (rabbit ear canker mite) and _Psoroptes_ _ovis_ (sheep scab mite).

The active compound may be used in the treatment of acarids with or without an appropriate carrier. When a carrier is used it will be an appropriate inert carrier such as corn oil or, preferably, an aqueous liquid. Powder inert carriers may be used if the composition is to be used in powder form. Typically, the active compound will be used at a concentration of not less than 1 ppm. Preferably, however, the active compound will be used as an aqueous solution at a concentration of not less than 5 ppm. The composition may, alternatively, be in the form of an emulsifiable concentrate in oil which, when mixed with water and a suitable emulsifier, forms an oil-in-water emulsion for use as a pesticide. The composition may, instead, be in the form of a solid wettable powder, such as may be obtained by using a powdered clay carrier. Compositions containing the compound 1 as an active ingredient may, if desired, additionally contain one or more other compounds, as is usual in the art, such as surface active agents, emulsifiers, antioxidants or other stabilisers, perfumes and colouring agents. The amounts of such components, when used, will be those conventionally used in the art.

### EXAMPLE 1

Determination of the efficacy of compound 1 vs. Psoroptes cuniculi, the ear canker mite of rabbits, by laboratory assay

*P.cuniculi* mites were collected the day before the bioassay and were harvested from artificially infested New Zealand White rabbits. The scabs and mites were collected into a clean, clear glass or plastic sample tube approximately 6.0 × 2.5 cm (height × diameter), secured with a tight fitting screw-topped lid.

Mites were kept cool during transportation.

The mites were stored overnight in a refrigerator at 4°C and were revived at approximately 22°C (room temperature) for 1 hour before commencing experimentation.

A fresh 96-well, flat bottomed, polystyrene tissue culture plate (with lid) was used for the bioassay (well diameter 6.4mm).

Each test material/concentration tested was referred to as "test material".

Two types of controls were tested, a positive control (usually the carrier of the test solution e.g., corn oil) and a negative control (usually water). Three wells, each containing 150µl of positive control and three wells, each containing 150µl of negative control were set-up using an Eppendorf microlite pipette. The same pipette tip was used for the three replicates of each control, but CLEAN tips had to be used for each different control.

Three replicate wells of 150µl of each test material to be bioassayed were set up. As with the controls, the same tip were used for each replicate, but a CLEAN tip was used for each different test material.

There were, therefore, three replicate wells of 150µl of each control and of each test material.

Adult female mites were used for the bioassay. They are the largest, darkest coloured mites, easily visible to the naked eye. Sex can be confirmed using a binocular zoom microscope (note the difference in leg length of the $4^{th}$ pair of legs - longer in the female; also note the difference in body size, the male is much smaller).

To avoid contamination of the mite collection tube with the test materials the following technique was used for each separate test material:

1. A small amount of scab and mites was transferred from the collection tube into the base of a 5.0cm diameter petri-dish.
2. The 5.0cm dish was placed into the base of a 9.0cm diameter petri-dish to give a moated "transfer dish".
3. Ten female mites were transferred to each of the replicate wells and the time was recorded.
4. The three replicate wells were checked under the binocular zoom microscope to ensure that they each had the correct number of live, active female mites.

The controls were set up first, followed by the lowest concentration test materials and ending with the highest for any test material. Once the wells were infested with mites the lid of the tissue culture plate was put in place to prevent any evaporation of liquid and escape of mites. All wells were then examined again under binocular zoom microscope and checked to ensure that they contained the correct number of mites.

The plates were then incubated at 22°C (room temperature) for 24 hours, with assessments carried out at the intervals of 1, 2, 3, 4, 5, 6 and 24 hours after the start of the test.

Mites were assessed and scored according to the following criteria, shown in Table 1:

Table 1

| Assessment criteria for mite bioassay | | |
|---|---|---|
| CRITERIA | STATUS | SCORE |
| Active movement of all limbs | LIVE | 1.0 |
| Weak movement of three or more limbs | MORIBUND | 0.5 |
| No movement at all | DEAD | 0.0 |

Using this method, each replicate received a score of between 0.0 and 10.0 at each assessment. The mean score was determined for the three replicates of each control or test material after 24 hours. This mean score was then used to determine the mean percentage mortality (mean %M), as follows:

$$\text{Mean \% M} = 100 - \left[\frac{\text{Observed score}}{\text{Max. score possible}} \times 100\right]$$

Control mortality should be corrected for by using Abbott's Formula:

$$\text{Adjusted \% C} = \left[\frac{\text{\%treatment - \% control}}{\text{100- \% control}} \times 100\right]$$

The results are shown in Table 2.

The compound supplied for testing was obtained from Maybridge Chemical Company, Trivillet, Tintagel, Cornwall, England and was used as a solution in water with an emulsifier, TRITON X100 ("TX 100").

Table 2

| Screen of compound 1 vs. Ear Canker Mite (ECM) | | | | | |
|---|---|---|---|---|---|
| Compound | 0.1 mg/ml in water +TX100, % Mortality (100ppm) | 0.025 mg/ml in water +TX100, % Mortality (25ppm) | 0.01 mg/ml in water +TX100, % Mortality (10ppm) | 0.005 mg/ml in water +TX100, % Mortality (5ppm) | 0.001 mg/ml in water +TX100, % Mortality (1ppm) |
| 1 | | | 100%,24 hrs | 100%,24 hrs | 30%, 24 hrs |

For comparison a commercial standard called "Bayticol" was included in the screen. "Bayticol" is a sheep dip containing the active ingredient flumethrin. Flumethrin technical material (TM) was bioassayed as a corn oil solution since it is insoluble in water. The results obtained from this test with the TM were 60% M at 4 mg $ml^{-1}$ (4000 ppm) a high concentration. The recommended use rate, however, when diluted as a dip is 44 ppm (0.044 mg $ml^{-1}$). During tests, it was discovered that a blank ECL (emulsifiable concentrate for livestock) formulation effected 100% M of ECM. This activity was traced to the benzoic acid in the formulation. The 0.044 mg $ml^{-1}$ (44 ppm) use rate was used as the commercial standard rate for comparison of the activity of compound 1 towards the Ear Canker Mite of rabbits.

At 0.05 mg $ml^{-1}$ compound 1 achieved 100% M in less than 1 hour. At 0.01 mg $ml^{-1}$, 0.005 mg $ml^{-1}$ and 0.001 mg $ml^{-1}$ compound 1 achieved 100% M, 100% M and 30% M, respectively indicating that the compound is particularly effective against ECM.

<u>EXAMPLE 2</u>

<u>Activity of compound 1 vs. *Psoroples ovis* (sheen scab mite)</u>

The activities of compound 1 (5 ppm) was investigated according to the following trials.

| | | | |
|---|---|---|---|
| 1. | Number of Samples | Test Samples | Ten (10) |
| | | Positive Control (Diazinon) | One (1) |
| | | Negative Control (Water) | One (1) |
| 2. | Number of Replicates | Three (3) | |
| 3. | Number of Mites Per Well | Between eight (8) and twelve (12) adult females | |
| 4. | Nature of Test Compound | Aqueous solutions. | |
| 5. | Amount of Test Solution | 3ml per sample | |
| 6. | Timing of Assessments | One hour | (+ 1h) |
| | | Three hours | (+ 3h) if required |
| | | Twenty four hours | (+ 24h) if required |

Two controls were set up, blank formulation and water.

An identical simultaneous screen was also run using the ear canker mite (*<u>Psoroptes</u> <u>cuniculi</u>*). A commercial standard for comparison purposes was DIAZINON (60% EC), tested at 10, 50, 75, 100 and 500 ppm for *<u>P. ovis</u>.*

<u>Results</u>

All results for *<u>P. ovis</u>* and *<u>P. cuniculi</u>* are shown together in Table 3 below. For comparison, the commercial standard Diazinon is normally made up at 400 ppm in the dip bath with a minimum maintenance level in the bath of 100-150 ppm.

Diazinon effected 100% M at 500 ppm, 76.1% M at 100 ppm, 91.4% M at 75 ppm, 59.7% M at 50 ppm and only 37.5% M at 10 ppm.

Compound 1 was active against both species of mite - giving similar results with both species.

Since this work was carried out, these two species have actually been reported as being the same species of mite *(Sheep scab conference, Cardiff, 1998*).

<u>Conclusion</u>

The work confirmed that the compound is specifically active against ectoparasitic mites, including the sheep scab mite (*P. ovis*) and the ear canker mite of rabbits (*P.cuniculi*).

Table 3

| Comparative activity of novel compounds vs. *P.ovis* and *P.cuniculi* | | | | | |
|---|---|---|---|---|---|
| COMPOUND | PPM | SPECIES | MEAN | % MORTALITY | |
| | | | 3 hours | 6 hours | 24 hours |
| 1 | 5 | *P.ovis* | 0.0 | 0.0 | 58.2 |
| 1 | 5 | *P.cuniculi* | 0.0 | 0.0 | 80.0 |
| | | | | | |
| BLANK | - | *P.ovis* | 0.0 | 0.0 | 10.4 |
| | - | *P.cuniculi* | 0.0 | 0.0 | 0.0 |
| WATER | - | *P.ovis* | 0.0 | 0.0 | 3.3 |
| | - | *P.cuniculi* | 0.0 | 0.0 | 0.0 |
| | | | | | |
| DIAZINON | 10 | *P.ovis* | 0.0 | 2.8 | 37.5 |
| DIAZINON | 50 | *P.ovis* | 0.0 | 2.8 | 59.7 |
| DIAZINON | 75 | *P.ovis* | 0.0 | 3.0 | 91.4 |
| DIAZINON | 100 | *P.ovis* | 0.0 | 0.0 | 76.1 |
| DIAZINON | 500 | *P.ovis* | 20.0 | 42.2 | 100.0 |

**Claims**

**1.** The use of 1-keto-3-(2-fur-2-ylethenyl)2-oxodihydroquinoline as an acaricide.

**2.** A method of combating acarids which comprises treating the acarids with an acaricidally-effective amount of 1-keto-3-(2-fur-2-ylethenyl)2-oxodihydroquinoline.

**3.** A method according to claim 2, wherein the acarids are selected from Psoroptes cuniculi and Psoroptes ovis.

**4.** An acaricidal composition comprising an inert carrier and, as active ingredient, 1-keto-3-(2-fur-2-ylethenyl)2-oxodihydroquinoline.

European Patent Office

# EUROPEAN SEARCH REPORT

Application Number
EP 00 30 3665

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DATABASE CHEMABS 'Online!<br>CHEMICAL ABSTRACTS SERVICE, COLUMBUS, OHIO, US;<br>F.C.WRIGHT: "Comparative efficacy of acaricides for the control of Psoroptes ovis (Hering) and P. cuniculi (Delafond)"<br>retrieved from STN-INTERNATIONAL, accession no. 94:115943 CA<br>XP002145134<br>* abstract *<br>& SOUTHWEST. ENTOMOL.,<br>vol. 5, no. 4, 1980, pages 222-225,<br>--- | 1-4 | A01N43/86 |
| A | DE 25 56 590 A (SHERWIN WILLIAMS CO)<br>2 September 1976 (1976-09-02)<br>* page 1, paragraph 2 - page 2, paragraph 2 *<br>--- | 1-4 | |
| A | EP 0 153 850 A (SAWAI SEIYAKU KK)<br>4 September 1985 (1985-09-04)<br>* page 4, paragraph 3 *<br>* page 15 - page 17 *<br>----- | 1-4 | TECHNICAL FIELDS SEARCHED (Int.Cl.7)<br>A01N |

The present search report has been drawn up for all claims

| Place of search | Date of completion of the search | Examiner |
|---|---|---|
| THE HAGUE | 30 August 2000 | Lamers, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 00 30 3665

This annex lists the patent family members relating to the patent documents cited in the above-mentioned European search report.
The members are as contained in the European Patent Office EDP file on
The European Patent Office is in no way liable for these particulars which are merely given for the purpose of information.

30-08-2000

| Patent document cited in search report | Publication date | Patent family member(s) | Publication date |
|---|---|---|---|
| DE 2556590 A | 02-09-1976 | US 3989698 A | 02-11-1976 |
| | | CA 1091668 A | 16-12-1980 |
| | | CH 629489 A | 30-04-1982 |
| | | FR 2301524 A | 17-09-1976 |
| | | GB 1520541 A | 09-08-1978 |
| | | IL 48559 A | 31-10-1978 |
| | | JP 51100086 A | 03-09-1976 |
| EP 0153850 A | 04-09-1985 | JP 1885981 C | 22-11-1994 |
| | | JP 6004584 B | 19-01-1994 |
| | | JP 60181072 A | 14-09-1985 |
| | | CA 1242731 A | 04-10-1988 |
| | | DE 3572976 D | 19-10-1989 |
| | | US 5006548 A | 09-04-1991 |

EPO FORM P0459

For more details about this annex : see Official Journal of the European Patent Office, No. 12/82